**Europäisches Patentamt**

**European Patent Office**

**Office européen des brevets**

(19)

(11) Veröffentlichungsnummer: **0 060 404**
**B1**

(12) **EUROPÄISCHE PATENTSCHRIFT**

(45) Veröffentlichungstag der Patentschrift:
05.12.84

(51) Int. Cl.³: **A 61 N 1/38**, H 02 H 9/02

(21) Anmeldenummer: **82101248.1**

(22) Anmeldetag: **18.02.82**

(54) Defibrillator.

(30) Priorität: **12.03.81 DE 3109489**

(43) Veröffentlichungstag der Anmeldung:
**22.09.82 Patentblatt 82/38**

(45) Bekanntmachung des Hinweises auf die Patenterteilung:
**05.12.84 Patentblatt 84/49**

(84) Benannte Vertragsstaaten:
**FR GB NL**

(56) Entgegenhaltungen:
**DE - A - 1 439 898**
**DE - A - 1 513 094**
**FR - A - 2 109 942**
**FR - A - 2 369 849**
**GB - A - 2 006 552**
**US - A - 3 983 461**

(73) Patentinhaber: **Hellige GmbH,**
**Postfach 728 Heinrich-von-Stephan-Strasse 4,**
**D-7800 Freiburg (DE)**

(72) Erfinder: **Huber, Peter, Schluchweg 8,**
**D-7835 Teningen 4 (DE)**

(74) Vertreter: **Patentanwälte TER MEER - MÜLLER -**
**STEINMEISTER, Triftstrasse 4, D-8000 München 22 (DE)**

## Beschreibung

Die Erfindung betrifft einen Defibrillator nach dem Oberbegriff des Patentanspruchs 1. Ein derartiger Defibrillator ist aus der DE-A-1 439 898 bekannt.

Defibrillatoren sind elektromedizinische Geräte, die zur Beseitigung von Herzkammerflimmern oder -flattern Verwendung finden.

Im Einsatzfall sind Defibrillatoren oftmals auf schnellstem Wege zum Patienten zu transportieren. Für den mobilen Einsatz sind handliche, leicht transportable Defibrillatoren besonders geeignet.

Der Erfindung liegt die Aufgabe zugrunde, einen Defibrillator mit geringem Gewicht und geringen äusseren Abmessungen zu schaffen, der darüber hinaus kostengünstig herstellbar sein soll.

Diese Aufgabe wird bei einem Defibrillator nach dem Oberbegriff des Patentanspruchs 1 durch den kennzeichnenden Teil dieses Patentanspruchs gelöst.

Vorteilhafte Weiterbildungen der Erfindung sind in den Unteransprüchen angegeben.

Bei dem erfindungsgemässen Defibrillator sind die bei Defibrillatoren bisheriger Bauart verwendete Induktivität zur Strombegrenzung im Entladestromkreis des Speicherkondensators und die Induktivität der Sekundärwicklung des Wandlertransformators im Ladestromkreis für den Speicherkondensator zu einem gemeinsamen Bauelement vereinigt.

Die Erfindung wird nachfolgend anhand von Ausführungsbeispielen unter Bezug auf die Zeichnung näher erläutert.

Es zeigen:

Fig. 1 den Aufbau des beanspruchten Defibrillators unter Verwendung eines Sperrwandlers und

Fig. 2 den Aufbau des Defibrillators unter Verwendung eines Summierwandlers.

Funktionell einander entsprechende Bauelemente sind mit gleichen Bezugszeichen gekennzeichnet.

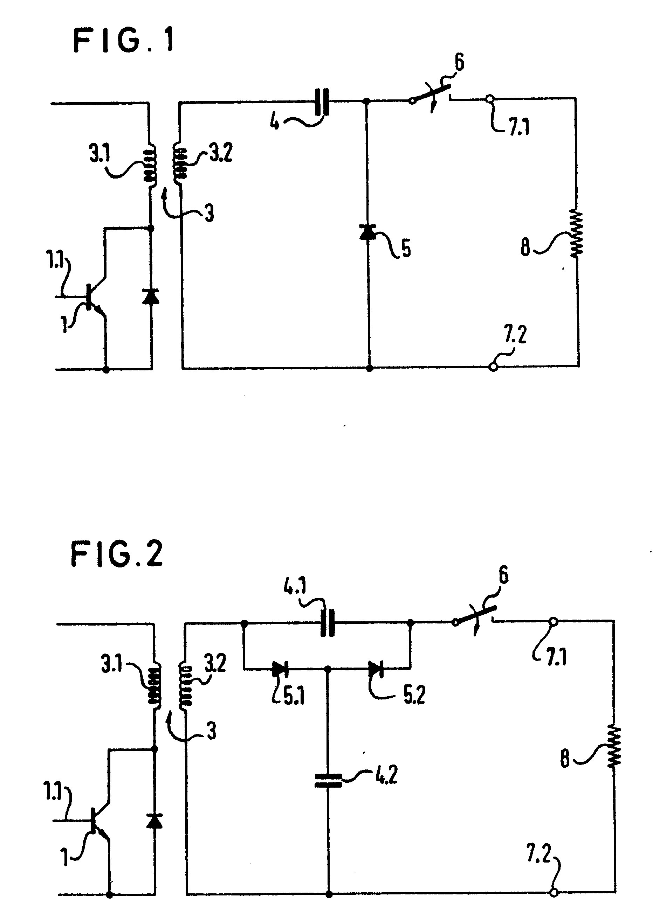

Die Fig. 1 zeigt schaltskizzenhaft den Lade- und Entladestromkreis eines Defibrillators. Aus einer nicht dargestellten Speisespannungsquelle fliesst über einen Transistor 1 ein pulsierender Strom durch die Primärwicklung 3.1 des Wandlertransformators 3. Über den Basisanschluss 1.1 des Transistors 1 wird der Strom durch die Primärwicklung 3.1 des Wandlertransformators 3 ein- und ausgeschaltet. Das Ein- und Ausschalten des Stroms erfolgt durch eine den Basisanschluss 1.1 des Transistors 1 beaufschlagende, in Fig. 1 nicht dargestellte Ansteuerelektronik. Während des gesamten Aufladezyklus ist ein Schalter 6 geöffnet. Entsprechend dem Übersetzungsverhältnis des Wandlertransformators 3 wird in dessen Sekundärwicklung 3.2 eine Spannung induziert, die im Sekundärkreis einen Strom hervorruft, der über eine Gleichrichterdiode 5 den Kondensator 4 auflädt.

Wird nach dem Ende der Aufladephase der Schalter 6 geschlossen, so entlädt sich der Kondensator 4 über Klemmen 7.1 und 7.2 über einen den Patientenstromkreis darstellenden Widerstand 8, wobei der Entladestrom zwangsläufig über die Sekundärwicklung 3.2 des Wandlertransformators 3 fliesst. Die Gleichrichterdiode 5 liegt während des Entladevorgangs in bezug auf den Patientenstromkreis in Sperrrichtung und beeinflusst damit den Entladevorgang nicht. Über die Induktivität der Sekundärwicklung 3.2 des Wandlertransformators 3 erfolgt eine Begrenzung des Entladestroms, der nach Schliessen des Schalters 6 zu fliessen beginnt. Eine Strombegrenzung im Entladestromkreis ist erforderlich, weil ansonsten wegen der zur erfolgreichen Defibrillation von Patienten aus medizinischer Sicht erforderlichen Niederohmigkeit des Patientenstromkreises und wegen der im Kondensator 4 aus gleichen Gründen gespeicherten grossen Energiemenge der Entladestrom auf unzulässig hohe Werte ansteigen würde und es somit bei der Defibrillation zu schweren Schäden kommen könnte.

In Fig. 1 ist der Lade- und Entladestromkreis eines Defibrillators unter Anwendung eines im Ladestromkreis nach dem Prinzip des Sperrwandlers arbeitenden Wandlertyps dargestellt, wobei durch die Induktivität der Sekundärwicklung 3.2 des Wandlertransformators eine Begrenzung des Entladestroms im Entladestromkreis erfolgt. Gemäss Fig. 2 arbeitet der Wandler im Ladestromkreis nach dem Prinzip des Summierwandlers. Auch bei Verwendung eines Summierwandlers dient die Induktivität der Sekundärwicklung 3.2 des Wandlertransformators 3 zur Strombegrenzung des Entladestroms. In diesem Fall sind zwei Kondensatoren 4.1 bzw. 4.2 und zwei Gleichrichterdioden 5.1 bzw. 5.2 erforderlich.

Gegenüber bekannten Defibrillatoren verringert sich die Anzahl der induktiven Bauelemente durch die Verwendung der Induktivität der Sekundärwicklung 3.2 des Wandlertransformators 3 zur Begrenzung des Entladestroms um ein wesentliches Bauteil. Induktive Bauelemente für hohe im Lade und Entladestromkreis von Defibrillatoren auftretende Ströme haben grosse Aussenabmessungen und ein grosses Gewicht, ihre Herstellung ist mit hohen Kosten verbunden.

Durch den neuen Aufbau des Lade- und Entladestromkreises des Defibrillators sind diese Geräte mit geringeren Aussenabmessungen, vermindertem Gewicht und kostengünstig herzustellen. Die erzielten Vorteile tragen zu einer Verbesserung von Defibrillatoren bei.

## Patentansprüche

1. Defibrillator mit einem Speicherkondensator (4), der von einer Impulsspannungsquelle über einen Ladestromkreis mit einem Wandlertransformator (3) aufgeladen und über einen Entladestromkreis mit einer Induktivität zur Strombegrenzung entladen wird, dadurch gekennzeichnet, dass die Induktivität der Sekundärwicklung (3.2) des Wandlertransformators (3) im Ladestromkreis zugleich als die Induktivität zur Strombegrenzung im Entladestromkreis dient.

2. Defibrillator nach Anspruch 1, dadurch gekennzeichnet, dass der Wandlertransformator (3) Teil eines Sperrwandlers ist.

3. Defibrillator nach Anspruch 1, dadurch gekennzeichnet, dass der Wandlertransformator (3) Teil eines Summierwandlers ist.

## Claims

1. A defibrillator comprising a storage capacitor (4) which is charged by a pulse-voltage source via a charging circuit including a converter transformer (3) and is discharged via a discharging circuit including an inductance for current-limiting, characterised in that the inductance of the secondary winding (3.2) of the converter transformer (3) in the charging circuit simultaneously serves as the inductance for current-limiting in the discharging circuit.

2. A defibrillator according to Claim 1, characterised in that the converter transformer (3) is a part of a blocking oscillator-type converter.

3. A defibrillator according to Claim 1, characterised in that the converter transformer (3) is a part of a summing-type converter.

## Revendications

1. Défibrillateur comprenant un condensateur d'accumulation (4) qui est chargé à partir d'une source de tension pulsatoire à travers un circuit de courant de charge avec un transformateur (3), et est déchargé à travers un circuit de courant de décharge avec une inductance pour limiter le courant, caractérisé par le fait que l'inductance de l'enroulement secondaire (3.2) du transformateur (3) sert dans le circuit de courant de charge, en même temps que l'inductance, à la limitation du courant dans le circuit de courant de décharge.

2. Défibrillateur selon la revendication 1, caractérisé par le fait que le trransformateur (3) est un élément d'un convertisseur à oscillateur bloqué.

3. Défibrillateur selon la revendication 1, caractérisé par le fait que le transformateur (3) est un élément d'un convertisseur additif.

## F I G . 1

## FIG.2